# EUROPEAN PATENT APPLICATION

(11) **EP 1 239 277 A1**
(43) Date of publication of application: **11.09.2002**
(21) Application number: 01105914.4
(22) Date of filing: 09.03.2001
(51) Int. Cl.: G01N 1/20, B01L 11/00

(54) **Measurement arrangement**

(71) Applicant: Infineon Technologies AG, 81669 München (DE)
(72) Inventor: Hertel, Martin, Dr., 07749 Jena (DE); Marx, Eckhard, 01471 Radeburg (DE); Schaef, Henry, 01561 Ebersbach-Naunhof (DE)
(74) Representative: Epping Hermann & Fischer

(57) **Abstract**

At least one - preferrably multiple - measurement sensors (1) and a vehicle (2) are combined to provide a mobile analysis of a chemical fluid used in a semiconductor fabrication facility. Peripheral equipment such as sets of plug connectors (3a, 3b) for transferring the chemical fluid from said processing tool (40), an electronic control system (5) with, e.g., a Laptop, a conduit system (8) with a pump system(9), and security means such as leakage detecting sensors (1a) and an automatic cleaning system are also provided on the vehicle (2). The measurement arrangement reduces bottleneck situations when qualifying processing tools (40) during fast ramp-up phases of semiconductor manufacturing facilities. The construction is based on PGVs or AGVs and allows a fast operation directly at the location of a processing tool (40). With the exception of the chemical connection, power supply or operator control, the measurement arrangement can operate fully autonomous.

## Description

The present invention relates to a measurement arrangement, for performing a measurement of at least one chemical or physical characteristic of a chemical fluid, which is used for processing a semiconductor device in a processing tool.

In semiconductor industry it is necessary to establish a fast ramp-up of new manufacturing facilities in order to retrieve a high return-on-invest (ROI). During this phase a large amount of test measurements have to be carried out for setting up and qualifying the new equipment, that has been installed in a corresponding manufacturing facility. To achieve this a variety of process analytic equipment is needed, e.g., to detect contaminating particles in a chemical fluid of a process bath, and to find the possible cause for this contamination.

Typically, the amount of process analytic equipment required for a manufacturing facility is usually planned to correlate with the needs during the production phase, which follows the ramp-up phase. Since a larger amount of process analytic equipment is needed in the ramp-up phase than in the production phase, a bottleneck during the ramp-up develops, which leads to a longer ramp-up phase, thereby reducing the return-on-invest of the fabrication.

Commonly, the chemical fluids of process bathes are controlled by process analytic equipment being implemented as stand-alone instruments. These stand-alone instruments are intended to monitor one specific process bath of a dedicated machine. In most cases those systems do not have a position near the location of the process machine, which is to be monitored. In particular in-situ to or inline measurements typically cannot be carried out with common stand-alone systems. Due to their weight and extent stand-alone instruments are on fixed positions in the fabrication facility and logistic and security efforts have to be undertaken in order to provide process bath measurements. Therefore, the footprint is disadvantageously increased and the process control duration prolonged.

In order to circumvent this problem the process analytic equipment in a fabrication facility, e.g. spectrometers, particle counters etc. can be embodied as laboratory instruments, which are more flexible. Unfortunately, strong efforts are as well necessary to perform measurements of the chemical fluids of, e.g., process bathes. Generally, the manufacturing process has to be stopped, and the processing tool is circumstantially opened in order to either carry out an in-situ measurement by contacting the measuring sensors of the laboratory instruments with the chemical fluid inside the manufacturing tool, or extract some chemical fluid from the tool for carrying out the measurement externally. In both cases crude security means are provided with the laboratory instruments. In particular the instruments have to be cleaned separately and the operator typically himself has to care about a possible leakage of the instruments or droplets remaining on the instrument surfaces.

Moreover, the portable laboratory instruments are generally neither temperature resistant nor unaffected by any acids. Further, they are as well dedicated to specific measurements, i.e. only a few specific parameters or characterictics of a chemical fluid can be recorded. The measurement of several parameters with just one instrument can generally not be carried out. Rather, more laboratory instruments are may be needed to provide a satisfactory process control. Since more operator staff is required to handle the different laboratory instruments while a considerable amount of production time is lost, costs are increasing and the return-on-invest of a fabrication facility decreases. The advantage of portability is counteracted by the disadvantage of modest functionality and security. Additionally, the quality of a measurement is moderate since in-situ methods in a real environment are not available.

It is therefore a primary objective of the present invention to increase the measurement quality of chemical fluids in a process bath in semiconductor manufacturing, and to reduce the costs particularly in the ramp-up phase of a fabrication facility for the qualifying and maintenance of manufacturing equipment.

The objective is solved by a measurement arrangement, for performing a measurement of at least one chemical or physical characteristic of a chemical fluid, which is used for processing a semiconductor device in a processing tool, comprising at least one measurement sensor for measuring said at least one characteristic of said chemical fluid, a first set of plug connectors for providing said chemical fluid from said processing tool to said at least one measurement sensor, a means for providing electrical power to said measurement arrangement, a first conduit system with a first pump system for circulating said chemical fluid through the at least one measurement sensor, an electronic control system for controlling said measurement sensor and the first pump system, and a vehicle for providing mobility to the arrangement, the vehicle being provided with spaces each for receiving said at least one measurement sensor, said electronic control system, said first set of plug connectors, said pump system, and said means for providing electrical power.

The vehicle, the measurement parts and the control parts being combined provide a mobile measurement arrangement, that advantageously allows to collect data of process bathes in the close facility of a corresponding manufacturing tool, which is to be qualified during ramp-up or is in system maintenance. Therefore, operators need not commute between different parts of the fab in order to perform the necessary measurements on the chemical fluids. The measurements can be performed in-situ and a feedback in case that problems with the process bath are recognized can be given instantaneously, e.g. process parameters of the manufacturing tool can be adjusted. in a given time interval on different manufacturing tools. Thus, the time needed for maintenance or qualifying of manufacturing tools is considerably shortened using the present invention. The production time is therefore increased and the costs are advantageously reduced.

According to the present invention a vehicle is provided with spaces or module frames, in which auch equipment can be placed, that is necessary to perform the chemical process analysis of process bathes of manufacturing tools. The placement can be performed e.g. by freely depositing the arrangement parts into corresponding module frames, thus being held just by gravity, or they are mounted fixed to the corresponding frames.

The chemical fluid under investigation can be anyone that is used for process bathes for processing semiconductor devices, preferably semiconductor wafers. In many cases these will be acids. Of these at least one chemical or physical characteristic, i.e. a parameter, is measured. Examples are the concentration, particle contamination, turbidity, density, viscosity, conductivity, acidity and the like of chemical fluids. To perform one of these measurements the arrangement is equipped with at least one measurement sensor. Thereby, it is possible that two different sensors measure one parameter or quantity serially or in parallel, or one measurement sensor is able to measure two different quantities etc. In order to provide highest flexibility a preferred embodiment comprises several measurement sensors for measuring all characteristics of the chemical fluid, that are needed to perform system maintenance.

The measurement arrangement is also provided with a (first) set of plug connectors by which a connection to the process tool can be easily and quickly accomplished. Preferably, the plug connectors are standardised in order to provide a connection to as many manufacturing tools as possible, thereby increasing the flexibility of the arrangement.

The set of plug connectors may comprise just one plug connector in case that the chemical fluid is transferred from the processing tool to the measurement sensors according to the present invention for performing the measurement, after which it is finally transferred back to the manufacturing tool via the just one plug connector.

More preferred is an implementation with two plug connectors, one for extracting the fluid from the manufacturing tool to the measurement sensor, and the other for recirculating the chemical fluid to the manufacturing tool. Thus, an inline measurement even during production is possible. It is also possible to provide more plug connectors, e.g. to provide connections to many tools obeying different plug connector standards.

For performing and controlling the measurement and for circulating the chemical fluid the measurement arrangement also comprises a means for providing electrical power. This is can either be an on-board accumulator making the arrangement independent from a local requirements, or a mains power supply with an electrical plug connector, which is plugged to the electrical network intervisinity of a manufacturing tool, which is to be measured.

The chemical fluid is circulated through a conduit system by means of a (first) pump system supplied with energy by the means for providing electrical power. The conduit system connects the plug connector via a pump or several pumps to the individual sensors. The pump system and the measurement sensors are controlled by an electronic control system, which preferably comprises a PC or labtop enabling an operator to perform the measurement. In a preferred embodiment, the plug connectors are also connected to the electronic control system in order to supply status information about whether a secure connection with a processing tool is established or not.

The vehicle providing the mobility to the arrangement can be similar to conventional personal or automatically guided vehicles (PGV or AGV). Thus, an operator can easily carry the measurement arrangement through the cleanroom area to the desired location at the manufacturing tool to be monitored.

In further aspects the weight and the dimension of the measurement arrangement are considered. In order to provide a measurement arrangement, that can easily be carried by an operator the weight is considered to be in the range of 20 kg and 150 kg, the length being less than 150 cm, the width being in the range from 30 cm up to 100 cm, and the height being in the range from 50 cm up to 180 cm. The arrangement then also fits to typical cleanroom area bay widths. This advantageously helps increasing the mobility, saving time and costs.

In a further aspect a further (second) set of plug connectors is implemented which provides a cleaning rinse or pressure gas for cleaning the measurement sensors and the conduit system after a measurement has been performed. Typically, high pressure gas is first lead through the conduit system and the sensors. Thereafter de-ionized clean water passes the conduit system and sensors. This second set of plug connectors is connected to the first conduit system by means of an additional conduit and pump system. It is supplied with valves for separating the conduit systems, and is being controlled by the electronic control system. This feature guarantees, that the measurement and the cleaning are not performed at the same time.

The main advantage is, that the mobile measurement arrangement can automatically and autonomously be cleaned on-board and possibly immediately after the measurement. Operators need not carry out cleaning efforts at different locations in a fab, which would tediously require additional footprint for cleaning equipment and time for further transport and remote cleaning. If a neighbouring process tool is to be monitored next, the measurement arrangement according to the present invention can be cleaned at its current location and then moved a few meters ahead for the following measurement. Therefore, the security of operators is enhanced, time is saved and costs are reduced.

In a further aspect a security means is provided to the measurement arrangement by a leakage collecting basin which extends on the vehicle below the conduit system in which the chemical fluid is transferred. Thus, if any acid leaks from the conduit system, it drops down into the basin and may be collected into a special acid container. The container may eventually be removed from the vehicle for disposing the acid elsewhere. A fluid detecting sensor is also mounted to the leakage collection basin, such that chemical fluid collecting in the basin can be detected and a warning signal is issued to the control system. A measurement just running can then be interrupted for security reasons and for impeding damage, thereby retaining the equipment quality.

In a further aspect the first set of plug connectors and the at least one measurement sensor are considered to be unaffected in their functionality by temperatures less than 473 K. This feature advantageously increases the variety of manufacturing tools and chemical fluids, by providing a broad range of temperatures, at which can be measured.

In a further aspect the first set of plug connectors and the measurement sensors are considered to be unaffected by acids. Materials are used in the sensors and the conduit systems, that are resistant against all in semiconductor manufacturing used acids, thereby also increasing the connectivity of the measurement arrangement according to the present invention to a variety of manufacturing tools.

In a further aspect the first conduit system is considered to comprise an isolation for retaining a nearly constant temperature of the chemical fluid. Therefore, equal process and measurement conditions are provided over a longer duration, which are the same as in the manufacturing tool itself, thus enabling a in-situ or inline measurement of chemical or physical parameters. For further retaining process conditions a heating and/or cooling unit can be implemented.

In a further aspect the security of the measurement arrangement is even further enhanced by considering the first set of plug connectors to be constructed with security means for protecting an operator from a physical contact with the chemical fluid, when the plug connectors are disconnected from the process tool. Droplets of acid that might have remained in the plug connectors are, e.g., enclosed inside the connector or are removed from the connector by electrical or mechanical means, which are supplied with the plug connectors.

In a further aspect the at least one measurement sensor is considered to be at least one of a particle counter for detecting particle contamination, a spectrometer with a light source and a light detector for measuring the concentration, turbidity or particle contamination in said chemical fluid, using either ultraviolet, visual or a near infrared light, an acoustic system for measuring the concentration, or density or viscosity, a measurement device for measuring the conductivity or acidity, or a refractometer for measuring the concentration in said chemical fluid. In still a further aspect it is also considered that any combination of the measurement sensors stated above can be used structured in the conduit system in parallel or serial. This means, that a conduit, in which a chemical fluid originating from the process tool is transferred via, e.g., a plug connector, can pass different or same types of measurement sensors in a series, or that this conduit may subdivide into two conduits supplying the two measurement sensors of the same or different kind with the chemical fluid in parallel. A combination of both is also possible.

In a further aspect the measurement arrangement is considered to comprise a data transfer unit for transferring data between the electronic control system and the processing tool. The data, that are to be transferred include information about the chemical structure of the fluid and other chemical or physical parameters. They either have to be known by the system prior to the measurement in order to adjust the sensor, pump and conduit units to these parameters, or they are specification limits, which are to be verified by the measurements.

The communication interface of the transferred data can either be put directly between the measurement arrangement and the processing tool, or can be performed via a factory wide communication system providing resource tracking data to any interested parties. Due to this the operator does not need to perform adjustment handling or to circumstantially search for specification data depending on the tool, which is to be controlled.

To increase the autonomy of the measurement arrangement a wireless communication interface for the data transfer between the measurement arrangement, i.e. the data transfer unit, and the processing tool or the factory wide communication system is considered in a further aspect.

A method using the measurement arrangement provided according to the present invention, by which the measurement of the at least one chemical or physical characteristic of the chemical fluid is performed, comprises the steps of moving the measurement arrangement from a first location to the location in front of a processing tool, connecting the plug connectors of the first set of plug connectors to said processing tool, circulating the chemical fluid from the processing tool through the plug connectors, performing the measurement of at least one chemical or physical characteristic of the chemical fluid, issuing an electronic or mechanical signal, after the measurement is finished, cleaning the measurement sensors and the conduit system in response to said signal, disconnecting the plug connectors from the processing tool, and moving said measurement arrangement to a third location.

Using the measurement arrangement according to the method of the present invention, the time needed to perform a measurment of a chemical fluid in a process bath and to monitor the process tool, when being in system maintenance can be efficiently reduced. Since the measurment arrangement is equipped with plug connectors, the operator's first task simply is to plug the connectors into the corresponding connectors of the processing tool. The tool neither has to be opened nor has the fluid to be transferred to a remote stand-alone instrument. Thus, a considerable amount of time is saved.

Next, the measurement is performed, with the advantage, that in case of a necessary feedback to the processing tool setup, this can be given immediately, because the measurement takes place locally. Again time is saved and the quality of the processing tool adjustment is improved, since the processing conditions of the chemical fluid are substantially retained as that in the process bath.

After the measurement is finished a signal is issued, that starts a cleaning of the conduit system, the pump system and the sensors. Preferrably, this is started automatically under control of the electronic control system in response to said signal. No efforts have to be undertaken to clean the arrangement parts externally using specific cleaning apparatus. This further improves the security of the operator and reduces time and costs.

In a further aspect of the present method the measurement arrangement is considered to be adjusted to measure a specific chemical fluid having a set of physical and chemical parameters provided by the process tool, e.g., via a host or data transfer unit. These parameters may be the process bath temperature, the chemical composition, the pressure, etc. The parameter settings of the measurement sensors are then adjusted to these conditions in order to achieve a high quality measurement. Most preferrably this part of the method is implemented as an automatic self-adjustment controlled by the electronic control system in response to a corresponding data transfer from the processing tool. The adjustment is carried out either prior to or during the measurement.

In a further aspect a self-diagnosis is performed by the measurement arrangement after or during the cleaning of the conduit systems with a cleaning rinse. The integrated sensors are used to examine the contamination of the rinse with particles indicating the current status and quality of the cleaning step. For this purpose a subset of the measurement sensors, which may be just one up to all of the measurement sensors, is used according to the specific adaption of the corresponding sensor.

Furthermore, a signal is issued in response to said cleaning rinse measurement, if the conduits and sensors of the measurement arrangement are clean and ready to perform a next measurement. If the conduit, etc. have for example a contaminating particle density, which is above a certain threshold, a corresponding signal can also be issued for continuing or repeating the cleaning step automatically. Waste of measuring time due to contaminated equipment is reduced, and expensive equipment is saved, because already integrated modules, i.e. measurement sensors, are used for the diagnosis of the arrangement (conduits, sensors, valves, connectors) itself.

Further advantageous features, aspects and details of the arrangement and the method according to the present invention are evident from the dependent claims.

The invention will be better understood by reference to the following description of embodiments of the invention taken in conjunction with the accompanying drawings, wherein
- figure 1: shows a top view of the measurement arrangement according to an embodiment of the present invention in a position of monitoring a process bath.
- figure 2: shows a schematical side view of the measurement arrangement according to an embodiment of the present invention.

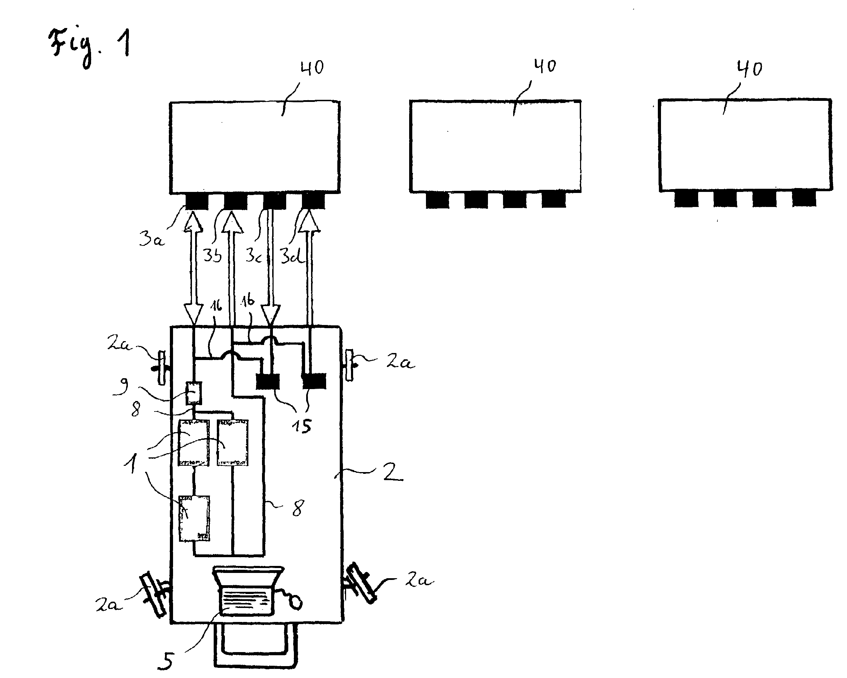

An embodiment of the measurement arrangement according to the present invention is shown in a top view in figure 1. In this schematical representation, a set of three measurement sensors 1 are mounted on a vehicle 2, which comprises four wheels 2a, two of them being steerable to enable an operator to easily move the vehicle 2 to a position directly in front of a processing tool 40, that is to be monitored.

As indicated by the arrows in figure 1 the arrangement comprises four plug connectors 3a - 3d, which are connected to the processing tool 40. A first set of plug connectors 3a, 3b serves for providing a circulation and recirculation flow of the chemical fluid under investigation from the processing tool to the measurement sensors 1 and back. I.e. an inline measurement of the process bath is enabled. While the plug connector 3b is just intended for refeeding the fluid back to the tool in order to reuse the fluid in realtime, which provides a main advantage of the present invention, the plug connector 3a on the one hand serves for extracting the chemical fluid from the tool 40, and on the other hand optionally can serve for both first extracting and second refeeding the chemical fluid from or to the processing tool, respectively. In the latter case an inline measurement is at least complicated, since there cannot be a continuous flow of the chemical fluid.

A pump system 9 generates the the flow of the chemical flow through the conduit system 8, which occasionally may subdivide into two parallel conduits, to which are attached the measurement sensors 1. Eventually, the two conduits merge again for providing one conduit for refeeding the fluid. Said three measurement sensors 1 in this embodiment are a visual and a UV-spectrometer and a particle counter.

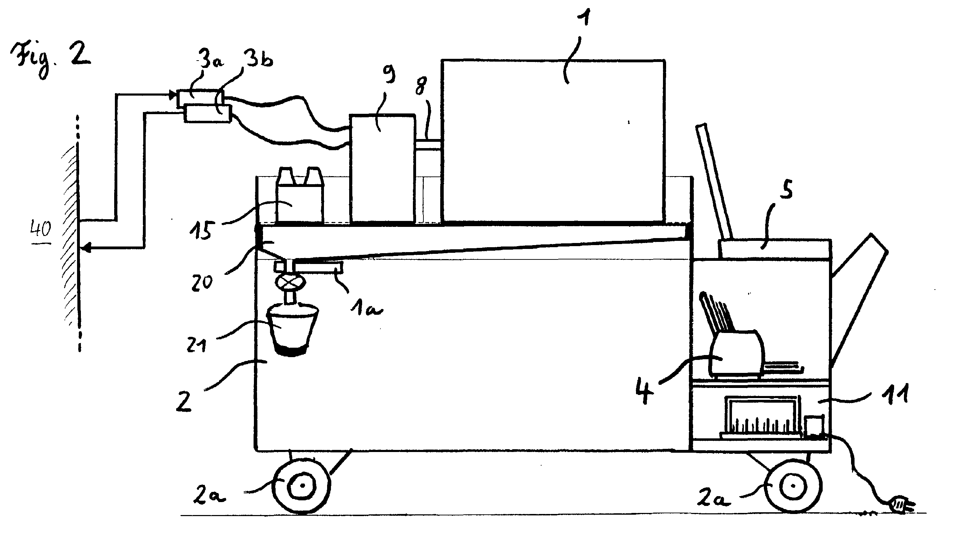

The measurement is controlled via a Laptop 5 as part of the electronic control system. The measured data are visualized in realtime, and process parameters of the arrangement such as circulation velocity and temperature (by means of, e.g., a heating unit) are monitored or adjusted for maintaining constant process conditions. Also, preferrably during inline measurements, the process tool setup can slightly be changed to visualize the effect on the process bath parameters in the measurement arrangement. The electronic connections of all system parts are not shown in the figures for clarity. The arrangement also comprises a printer 4 as shown in the embodiment of figure 2.

After the measurement is finished, the conduit system 8, the pump system 9 and the measurement sensors 1 are cleaned by means of the cleaning system 15, that also includes a pump system and a conduit system 16. Plug connectors 3c - 3d provide the source and drain of cleaning material, respectively. First the acid is blown out off the conduits by Nitride pressure gas, after which a cleaning rinse is led through said conduits. Valves controlled by the electronic control system 5 provide for a separation of the cleaning rinse and the chemical fluid., i.e. the measurement and the cleaning.

In the side view of an embodiment of the present invention a leakage collecting basin 20 is mounted to the vehicle, which extends below the conduits, sensors and pumps of the arrangement. A leakage detecting sensor 1a is mounted at the spout of the basin. Below the spout, a removable container for leakage fluid 21 can receive the acid fluid. Leakage of acid therefore can be detected and removed, while a signal, that is issued by the leakage detecting sensor 1a and received by the electronic control system eventually result in security actions undertaken by the operator in response the signalled leakage.

The arrangement according to the embodiment also comprises a mains power supply and an accumulator 11. In figure 2, the electric plug connector is not plugged in, and the electric power supply is maintained by the accumulator.

The embodiment according to figures 1 and 2 has a weight of 100 kg, a length of 120 cm, a width of 50 cm and a height of 70 cm.

List of reference numerals
- 1: measurement sensor
- 1a: leakage detecting sensor
- 2: vehicle
- 2a: wheel
- 3a: plug connector for chemical fluid (in/out)
- 3b: plug connector for chemical fluid (out)
- 3c: plug connector for cleaning rinse / pressure gas (in)
- 3d: plug connector for cleaning rinse / pressure gas (out)
- 4: printer
- 5: electronic control system
- 7: data transfer unit
- 8: first conduit system
- 9: first pump system
- 11: means for providing power supply, mains power supply
- 15: cleaning system with pump system
- 16: second conduit system
- 20: leakage collecting basin
- 21: removable container for leakage fluid
- 40: processing tool

## Claims

1. Measurement arrangement, for performing a measurement of at least one chemical or physical characteristic of a chemical fluid, which is used for processing a semiconductor device in a processing tool (40), comprising:
- at least one measurement sensor (1) for measuring said at least one characteristic of said chemical fluid,
- a first set of plug connectors (3a, 3b) for providing said chemical fluid from said processing tool to said at least one measurement sensor (1),
- a means for providing electrical power (11) to said measurement arrangement,
- a first conduit system (8) with a first pump system (9) for circulating said chemical fluid through the at least one measurement sensor (1),
- an electronic control system (5) for controlling said measurement sensor (1),
- a vehicle (2) for providing mobility to the arrangement, the vehicle being provided with spaces each for receiving
a) said at least one measurement sensor (1),
b) said electronic control system (5),
c) said set of plug connectors (3),
d) said first conduit system (8) with a first pump system (9),
e) said means for providing electrical power (11).

2. Arrangement according to claim 1,
**characterised by**
a second set of plug connectors (3c, 3d) and a second conduit system (16) with a second pump system to enable the cleaning of the first conduit system (8) and of the at least one measurement sensor (1) by means of pressure gas or cleaning rinse.

3. Arrangement according to anyone of claims 1 to 2,
**characterised in that**
said measurement arrangement has
- a width of more than 300 millimeters and a width less than 1000 millimeters,
- a length less than 1500 millimeters,
- a heigth of more than 500 millimeters and a heigth less than 1800 millimeters.

4. Arrangement according to anyone of claims 1 to 3,
**characterised by**
a leakage collecting basin (20), which is mounted on the vehicle (2) below the first conduit system (8), for collecting chemical fluid leaking from said first conduit system (8), and
a fluid detecting sensor (1a) for detecting chemical fluid collecting in said basin (20).

5. Arrangement according to anyone of claims 1 to 4,
**characterised by**
a drive, a brake and at least one steerable wheel (2a) mounted to the vehicle (2), for providing a self-powered movement of the arrangement.

6. Arrangement according to anyone of claims 2 to 5,
**characterised in that**
said means for providing electrical power (11) to said measurement arrangement comprises an accumulator.

7. Arrangement according to anyone of claims 1 to 6,
**characterised in that**
said first set of plug connectors (3a, 3b) and the at least one measurement sensor (1) are unaffected in their function by temperatures less than 473 Kelvin.

8. Arrangement according to anyone of claims 1 to 7,
**characterised in that**
said first set of plug connectors (3a, 3b) and the at least one measurement sensor (1) are acid-resistant.

9. Arrangement according to anyone of claims 1 to 8,
**characterised in that**
said first conduit system (8) comprises an isolation, such that said chemical fluid circulating retains a nearly constant temperature.

10. Arrangement according to anyone of claims 1 to 9,
**characterised in that**
each of the plug connectors of said first set of plug connectors (3a, 3b) comprises a leakage protecting means for protecting the environment from a physical contact with said chemical fluid when being disconnected from said process tool (40).

11. Arrangement according to anyone of claims 1 to 10,
**characterised in that**
said at least one measurement sensor (1) is at least one of:
- a particle counter for detecting particle contamination in said chemical fluid,
- a spectrometer with light source and light detector for measuring the concentration, or the turbidity, or particle contamination in said chemical fluid, using either ultraviolet, or visual, or near infrared light,
- an accoustic system for measuring the concentration, or the density, or the viscosity in said chemical fluid,
- a measurement device for measuring the conductivity or the acidity of said chemical fluid,
- a refractometer for measuring the concentration in said chemical fluid.

12. Arrangement according to anyone of claims 11,
**characterised by**
at least two of said measurement sensors (1),
which are a combination of at least two of said particle counter, or said spectrometer, or said accoustic system, or said measurement device for measuring the conductivity or the acidity, or said refractometer, and
which are attached to the conduit system (8) on parallel and/or serial conduits.

13. Arrangement according to anyone of claims 1 to 12,
**characterised by**
a printer (4) connected to the electronic control system (5), said vehicle (2) being provided with space for receiving said printer (4).

14. Arrangement according to anyone of claims 1 to 13,
**characterised by**
a data transfer unit (7) for transferring data between the electronic control system (5) and the processing tool (40), said vehicle (2) being provided with space for receiving said data transfer unit (7).

15. Arrangement according to claim 14,
**characterised in that**
said data transfer unit (7) comprises a wireless communication interface for data transfer.

16. Arrangement according to anyone of claims 1 to 15,
**characterised by**
a means for docking said vehicle at a location in a fabrication system.

17. Arrangement according to anyone of claims 1 to 16,
**characterised in that**
the measurement arrangement has a total weight of more than 20 kg and less than 150 kg.

18. Arrangement according to anyone of claims 7 to 17,
**characterised by**
at least two plug connectors (3a, 3b) of said first set of plug connectors each for receiving and recirculating said chemical fluid between said processing tool (40) and said measurement arrangement.

19. Arrangement according to anyone of claims 1 to 15,
**characterised in that**
said vehicle (2) comprises at least one wheel (2a) for rolling the vehicle between two locations.

20. Method for performing a measurement of at least one chemical or physical characteristic of a chemical fluid, which is used for processing a semiconductor device in a processing tool (40), using the measurement arrangement according to anyone of claims 1 to 19, comprising the steps of:
- moving the measurement arrangement from a first location to the location in front of a processing tool (40),
- connecting the at least one plug connector of the first set of plug connectors (3a, 3b) to said processing tool (40),
- circulating the chemical fluid from the processing tool (40) through the at least one plug connector,
- performing the measurement of at least one chemical or physical characteristic of a chemical fluid using the at least one measurement sensor (1),
- issuing an electronic or mechanical signal, when the measurement is finished,
- cleaning the measurement sensors (1) and the conduit system (8) in response to said signal,
- disconnecting the plug connectors from the processing tool (40),
- moving said measurement arrangement to a third location.

21. Method according to claim 20,
**characterised by**
adjusting measurement parameter settings of at least one measurement sensor (1) in response to a data tranfer from the processing tool (40) containing physical or chemical information.

22. Method according to claim 21,
**characterised by**
controlling the flow of said chemical fluid through conduits of said first conduit system (8) by opening or closing valves by means of said electronic control system (5) for selecting one of the at least one measurement sensor (1) in response to said data transfer.

23. Method according to anyone of claims 20 to 21,
**characterised by**
issuing a warning signal, if the fluid detecting sensor (1a) detects chemical fluid in the leakage collecting basin (20).

24. Method according to anyone of claims 20 to 23,
**characterised by**
recirculating the chemical fluid back to said processing tool (40), while performing said measurement.

25. Method according to anyone of claims 20 to 24,
**characterised by**
performing the measurement of at least two of the chemical or physical characteristics of said chemical fluid at the same time.

26. Method according to anyone of claims 20 to 25,
**characterised by**
measuring the particle contamination in said cleaning rinse during or after the cleaning of said first conduit system (8) and the at least one measurement sensor (1) using a subset of the at least one measurement sensor (1).

27. Method according to claim 26,
**characterised by**
issuing a signal in response to said cleaning rinse measurement, if the conduits and sensors of the measurement arrangement are clean and ready to perform a next measurement.
